Europäisches Patentamt

(19) European Patent Office

Office européen des brevets

(11) Publication number: **0 322 213 B1**

# EUROPEAN PATENT SPECIFICATION

(12)

(45) Date of publication of patent specification: **13.04.94**   (51) Int. Cl.⁵: **C12P 7/64**, C12N 11/08,
//(C12P7/64,C12N9:20)

(21) Application number: **88312116.2**

(22) Date of filing: **21.12.88**

The file contains technical information submitted after the application was filed and not included in this specification

(54) Process for the preparation of fatty acid esters.

(30) Priority: **22.12.87 GB 8729890**

(43) Date of publication of application:
**28.06.89 Bulletin 89/26**

(45) Publication of the grant of the patent:
**13.04.94 Bulletin 94/15**

(84) Designated Contracting States:
**AT BE CH DE ES FR GB GR IT LI NL SE**

(56) References cited:
**EP-A- 0 195 311**
**US-A- 4 629 742**

(73) Proprietor: **UNILEVER PLC**
**Unilever House**
**Blackfriars**
**P.O. Box 68**
**London EC4P 4BO(GB)**

(84) Designated Contracting States:
**GB**

(73) Proprietor: **UNILEVER N.V.**
**Weena 455**
**NL-3013 AL Rotterdam(NL)**

(84) Designated Contracting States:

BE CH DE ES FR GR IT LI NL SE AT

(72) Inventor: **Macrae, Alasdair Robin**
**Ivy Cottage**
**Newton Blossomville Bedford MK43 8AN(GB)**
Inventor: **Bosley, John Anthony**
**43 Kettering Road**
**Islip**
**Kettering Northants. NN14 3JT(GB)**
Inventor: **Peilow, Alan David**
**17 Lancaster Close**
**Wollaston Northants. NN9 7PD(GB)**

(74) Representative: **Dries, Antonius Johannes Maria et al**
**Unilever N.V. Patent Division**
**P.O. Box 137**
**NL-3130 AC Vlaardingen (NL)**

Rank Xerox (UK) Business Services
(3.10/3.09/3.3.3)

**Description**

This invention relates to processes for the preparation of fatty acid esters or for the re-arrangement of fats and vegetable oils, wherein reactants providing fatty acid and alcohol residues are reacted under the influence of lipases as catalysts.

In the re-arrangement of fats and vegetable oils their fatty acid constituents are re-arranged to a greater or lesser extent on the triglycerides of which the fats and oils are largely composed, according to the catalyst selected for the reaction. Under the influence of alkali metals, their hydroxides and alkoxides, the re-arrangement is invariably random. In a more recently developed method described in US-A-4275081 using lipase catalysts, the re-arrangement may be random or selective according to the lipase selected and additional fatty acid radicals present as free acid or alkyl esters in the reactant compositions may participate. As described in this and other references, the enzyme is preferably fixed on a inert support material which may be inorganic and/or organic and the reaction may be operated batchwise or continuously, in the presence or absence of solvents for the reactant and at temperatures and other conditions at which the reactants are in liquid, homogeneous, water-immiscible phase and the catalyst is active.

Whereas re-arrangement processes under the influence of alkali metal catalysts must be conducted with water rigorously excluded in order to avoid side reaction with the production of soap and loss of catalytic activity, enzyme catalysts require a small amount of water for activation throughout the reaction. Sufficient water may be provided in feedstock in the case of continuous reactions in which the enzyme is fixed on a catalyst support in a bed through which the feedstock passes, the amount of water usually being less than 1% and even as little as 0.1% by weight of the feedstock. In the presence of substantially greater amounts of water than this, the catalyst remains active but the reaction is directed to the production of hydrolysis products with loss of triglycerides. On the other hand, attempts to minimise the amount of water present results in decrease of catalyst activity.

Similar considerations apply, in regard to the limited amount of water permitted, in esterification reactions carried out under the influence as catalyst of lipase enzymes (including alcoholysis reactions) in which the alcohol residue of an ester - which may of course be a glyceride ester such as a triglyceride - is replaced by that of another alcohol. These reactions share with re-arrangement processes the necessity for maintaining the presence of water in very small amounts in the reaction system in order to avoid the production of undesired hydrolysis products.

In re-arrangement processes and related processes described which are carried out in the presence of very limited amounts of water under the influence as catalyst of a supported enzyme lipase, the enzyme is deposited on support material offering a large surface area which may be inorganic, for example silica and its derivatives including in particular Celite (trade name for diatomaceous earth), or organic, for example ionic exchange resins having a macroreticular structure in the pores of which the enzyme can be accommodated. Hitherto in these reactions the attachment of the enzyme to the support material has been effected physically by precipitating the enzyme on a hydrophilic support surface, or as described in EP-A-147914 by the application of an organometallic coupling compound to bind the enzyme chemically to the carrier while forming a hydrophobic environment, or by ionic attachment to ion exchange material as described in EP-A-140542. Ionic attachment of enzymes to modified polystyrene polymer is described in US-A-4551482. Preparation of a polymer surface, which is initially hydrophobic, by soaking in a dilute solution of a long-chain cationic surfactant, prior to enzyme immobilization is described in US-A-4539294.

The object of the invention is to provide improved processes for preparation of fatty acid esters.

It has now been found that above objections are fulfilled by a process for the preparation of fatty acid esters, or for the re-arrangement of fats and vegetable oils, wherein reactants providing fatty acid and alcohol residues are reacted in a substantially non-aqueous, homogeneous liquid phase containing maximum 1 wt% of water in the presence of a supported lipase catalyst obtained by physically attaching the lipase by adsorption to the support material, characterized in that the support for the lipase catalyst is selected from the group consisting of 1) polyalkylenes, selected from the group consisting of homo- or copolymers of ethylene, styrene, butadiene or isoprene and homopolymers of propylene and 2) inorganic porous materials whose surfaces have been treated to render them hydrophobic, which support material has an average pore size from 0.05-5 $\mu$m.

Therefore, the reaction of the invention takes place in a substantially non-aqueous phase, however, some water is required to be associated with the enzyme. This amount of water is almost immeasurably small. For example saturation of the non-aqueous phase with water is sufficient; 50% or more of saturation can also be applied. No aqueous phase is present.

The support materials must be made porous and this may be effected by the application of blowing agents during polymerisation. Inorganic porous materials e.g. glass and refractory material e.g. silicas whose surface has been rendered hydrophobic by e.g. silane treatment, are also suitable. To achieve good immobilization of the enzyme, the average pore size of support material is not less than 50 nm, particularly 0.05-5 μm. By average pore size we mean the mean pore diameter as measured by the standard techniques of mercury porosimetry. Particle size of suitable material is preferably 0.01-5 mm, especially 0.1-1.5 mm for polyolefins and 0.5-3 mm for refractory materials such as silica.

Enzymes supported on hydrophobic material are described in US-A-4629742 where they are used for hydrolysis of fats. Particularly, useful synthetic polymers in the present invention are polystyrenes, e.g. as prepared by the high internal phase emulsion technique described in EP-A-60138

EP 195311 discloses a process, wherein sterol esters or specific aliphatic alcohol esters of fatty acids can be prepared by an enzymic process. The enzymic material is supported.

However, the amount of water applied in above system is considerable.

The supported enzyme can be prepared according to four methods, including an adsorption method. However, it is disclosed that the adsorption method can only be applied on hydrophilic supports, which have been modified, such as modified polysaccharides.

It is also disclosed that inorganic supports could be applied in the adsorption method. However, the inorganic materials mentioned are all hydrophilic materials. We have found that these support materials are unsuitable for the purpose of our invention.

Preferably, the support material comprises a porous polymeric material having a total pore volume of at least 50% and comprising a plurality of voids interconnected by holes, the voids having an average diameter within the range of from 1 to 150 μm. The total pore volume is more preferably at least 75%, e.g. 90% or more.

Further details concerning suitable porous materials for use in the present invention can be found in the above mentioned EP-A-60138 and additionally in EP-A-105634, EP-A-156541, EP-A-157504, GB-A-2155481, EP-A-200528, EP-A-223574, EP-A-239360, EP-A-240342, EP-A-264268, EP-A-289238, EP-A-288310 and EP-A-299 762.

Each of these patent specifications describes a porous material which conforms to the present porosity requirements. In all cases the material can be made by polymerising starting materials in the form of a high internal phase emulsion which can either be a water-in-oil emulsion, an oil-in-water emulsion or an emulsion formed between any two sufficiently immiscible solutions. By "water" we means aqueous based and by "oil" we mean immiscible with aqueous system. The materials described in the different specifications differ in their starting materials and/or end properties.

Japanese patent publication J84091883 describes a preparation of supported enzyme by contacting an enzyme solution with styrene or methacrylic acid esters. Styrene polymers are proposed as enzyme support material in Russian patent specification SU 804647. Hoq et al (JAOCS 61, (4) April 1984, pp. 776-81) describes glyceride synthesis under the influence as catalyst of lipase immobilised on a hydrophobic porous support material e .g. polypropylene , in a heterogeneous reaction mass comprising immiscible aqueous and non-aqueous phases.

Alternative supports useful in the invention are inorganic refractory materials e.g. silica or glass. Such material must have been treated in order to make their surfaces hydrophobic e.g. by a treatment with a hydrophobic organic silica polymer coating.

Suitable enzymes for use in the invention include lipases from lipase species of the genuses Mucor, Aspergillus, Rhizopus, Pseudomonas, Candida, Humicola, Thermomyces and Penicillium.

The reactants used in the invention to provide fatty acid and alcohol residues preferably comprise glyceride esters, e.g. at least one of a fat and an oil.

One preferred reactant system comprises glyceride esters together with at least one free fatty acid or alkyl ester thereof.

EXAMPLES

To illustrate the invention, there are given below a number of Examples.

In these Examles, a variety of supports for the lipases is used. Their characterization is as follows:

EP-100, EP-400

Obtainable from Enka, Netherlands

Type: macroporous particles of polypropylene (EP-100, EP-400) having large cells (voids) interconnected by holes.

EP 0 322 213 B1

Trade Name: Accurel

|  | EP-100 | EP-40 |
|---|---|---|
| Particle size (mm) | 0.2-0.4 | 0.2-0.4 |
| Pore volume (ml/g) | 3.2 | 0.6 |
| Surface area (m˜/g) | 92 | 2 |
| Bulk density (g/ml) | 0.15 | 0.2 |
| Mean pore diameter (nm) | 139 | 1207 |
| Cell size (pm) | 0.5-5.0 | 1.0-5. |
| Hole size (rm) | 0.05-0.5 | 0.1-0.5 |
| Void volume (%) | 75 | 75 |

S. 980G

Obtainable from Shell
Type: porous silica spherical particles

| Particle size | 1.5 mm |
|---|---|
| Pore volume | 1.2 ml/g |
| Surface area | 63 $m^2$/g |
| Bulk density | 0.4 g/ml |
| Mean pore diameter | 60 nm |

5693-93, 78-7300

Obtainable from National Starch & Chemical Ltd, UK

Type: macroporous polystyrene particles having large cells (voids) interconnected by holes, made by emulsion polymerization with the monomers in the external phase and a high internal phase volume. Total pore volume is greater than 90%.

|  | 5693-93 | 78-7300 |
|---|---|---|
| Surface area (BET) | 11.3 $m^2$/g | 6.3 m g |
| Pore size distribution | 500-7000 nm | 1000-6000 nm |
| Mean pore diameter | 1686 nm | 3360 nm |
| Total pore volume | 4.7 ml/g | 5.1 ml1/g |

AMBERLITE XE-305

Obtainable from Rohm & Haas
Type: macroporous polystyrene particles

| Surface area (BET) | 43.7 m /g |
|---|---|
| Pore size distribution | 30-200 nm |
| Mean pore diameter | 55 nm |

4

PG-2000-120

Obtainable from Sigma Chemical Co. Ltd.
Type: controlled-pore glass beads

| | |
|---|---|
| Surface area | 11.1 m$^2$/g |
| Mean pore diameter | 205.9 nm |

## EXAMPLE 1

i) Immobilization

8 grams of EP 100 was added to 100 ml ethanol and stirred vigorously to ensure that all powder was wetted. 60 ml excess ethanol was decanted and 200 ml of 0.1M phosphate buffer pH 7.0 was added and stirred to ensure complete mixing. Excess buffer was removed by gentle suction on a sintered glass funnel. The wetted powder was then added to 100 ml 0.1M phosphate pH 7.0 containing 10.0g 1,3 selective Mucor miehei lipase powder (obtainable from Novo, Denmark) and stirred slowly. The adsorption of lipase by the polymer was assessed by loss of lipase activity from solution. After 28 hours the support had adsorbed 93% of the lipase activity, giving a theoretical loading of 16,000 LU/g (Lipase units/g). The catalyst was removed by filtration, washed twice with 200 ml of 0.1M phosphate pH 7.0 and dried under vacuum at room temperature.

ii) Interesterification

2.0g of catalyst was packed in a column 15 mm in diameter, together with 4g wet ID silica gel (obtainable from Crosfields, UK) containing 3.2g water as a pre-column. Water-saturated feedstock comprising 1 part high oleate sunflower oil, 0.7 parts lauric acid and 4 parts petroleum ether (BP 100-120'') by weight, was pumped through the column at a flow rate of 25.0 ml/hour and the column maintained at 50°C using a water jacket. The amount of lauric acid incorporated into the triglyceride was determined by FAME/GC analysis (Fatty Acid Methyl Ester/Gas Chromatography analysis).

A control experiment was carried out under similar conditions, using a proprietary catalyst "Lipozyme" (Novo) based on Mucor miehei lipase on an ion exchange resin (hydrophilic). The results are compared in the Table 1 which gives the triglyceride analysis of the feedstock oil and the product of reaction.

TABLE 1

| fatty acid in triglycerides | Feedstock oil | Product EP100 | Lipozyme |
|---|---|---|---|
| C10:0 | 0.0 | 0.0 | 0.3 |
| C12:0 | 0.0 | 26.2 | 17.2 |
| C14:0 | 0.0 | 0.0 | 0.6 |
| C16:0 | 3.7 | 1.6 | 2.6 |
| C18:0 | 4.7 | 2.2 | 3.6 |
| C18:1 | 84.9 | 65.5 | 69.3 |
| C18:2 | 6.3 | 4.5 | 6.0 |
| C20:0 | 0.4 | 0.0 | 0.4 |

## EXAMPLE 2

Example 1 was repeated twice using as catalyst supports EP100 and EP4OO powders respectively and with a different lipase. The lipase was Rhizopus niveus (Amano N obtainable from Armano, Japan). Preparation was as described in Example 1 except that theoretical lipase loadings of 10,000 Lu/g were obtained. The results are shown in Table 2.

TABLE 2

| Fatty acid in triglycerides | Feedstock oil | Product | |
|---|---|---|---|
| | | EP100 | EP400 |
| C10:0 | 0.0 | 0.0 | 0.0 |
| C12:0 | 0.0 | 28.5 | 29.0 |
| C14:0 | 0.0 | 0.9 | 0.5 |
| C16:0 | 3.7 | 1.6 | 1.6 |
| C18:0 | 4.7 | 2.2 | 2.0 |
| C18:1 | 84.9 | 62.3 | 61.6 |
| C18:2 | 6.3 | 4.5 | 4.4 |
| C20:0 | 0.4 | 0.0 | 0.0 |

From Table 1 it is clear that replacement by lauric acid is substantially greater with hydrophobic catalyst support, compared with the effect of the hydrophilic material, and from Table 2 that good results are also obtained with other supports and another enzyme.

EXAMPLE 3

i) Support preparation and immobilization of enzyme

5g of silica spheres S.980G were rendered hydrophobic by shaking gently in 100 ml 1,1,1-trichloroethane containing 10g dimethyl dichlorosilane for ½ hour. After filtering and washing twice with 100 ml of the trichloroethane the spheres were dried at room temperature under reduced pressure. The silanised spheres were gently shaken in 50 ml of ethanol to ensure complete wetting and 100 ml 0.1M sodium phosphate buffer at pH7 was added with gentle shaking to ensure complete mixing. After decanting excess buffer, 50 ml aqueous lipase solution (Mucor miehei at 1,000 Lu/ml activity) was added and shaken with the spheres for 16 hours. The spheres were then filtered, washed twice with a buffer solution and dried as before.

ii) Interesterification

The resulting catalyst was used in the re-arrangement method described in Example 1, using as feedstock 0.53 parts lauric acid and 0.27 parts myristic acid per part of sunflower oil. Triglyceride analysis results appear in Table 3.

TABLE 3

| Fatty acid in triglycerides | Feedstock oil | Product 5980G |
|---|---|---|
| C10.0 | 0.0 | 0.1 |
| C12.0 | 0.0 | 14.6 |
| C14.0 | 0.0 | 6.6 |
| C16.0 | 3.7 | 2.3 |
| C18.0 | 4.7 | 3.1 |
| C18.1 | 84.9 | 68.4 |
| C18.2 | 6.3 | 4.9 |
| C20.0 | 0.4 | 0.0 |

The results in Table 3 show that excellent substitution of the unsaturated acids in the sunflower oil by the lauric and myristic acids takes place under the influence of the supported enzyme.

EP 0 322 213 B1

EXAMPLE 4

i) Preparation of porous support

A high internal phase water-in-oil emulsion was formed in which the oil phase comprised styrene, divinylbenzene and surfactant Span 80 (4.42, 0.44 and 0.88 kg respectively) and the aqueous phase contained water, sodium persulphate and calcium chloride (44.25, 0.097 and 9.0 kg respectively). Span 80 is sorbitan mono-oleate. The emulsion was subjected to 30 minutes post-formation sheer at 85 rpm. Polymerisation was subsequently carried out at 60°C over 40 hours. The resulting polymer was milled, then sieved to the desired particle size and soxhlet-extracted with isopropanol to remove Span 80 followed by washing with de-ionised water and finally dried. Microscope examination indicated that the polystyrene material had a porosity of 90%, a cell (void) size of at least 30 $\mu$m, an interconnecting hole size of at least 10 $\mu$M and a particle size of 1.0 to 2.0 mm.

ii) Immobilization

Polymer (2.0g) was placed in absolute ethanol (135 ml) to fully wet the polymer. Excess ethanol (120 ml) was decanted and 0.1M sodium phosphate buffer pH 7.0 (200 ml) was added and gently stirred for 2 hours to ensure complete mixing. Excess solution was decanted (195 ml) and a further quantity of buffer (200 ml) added and stirred for 10 minutes. Excess solution was again decanted (200 ml). Lipase solution (100 ml) 0.1M sodium phosphate pH 7 containing 7.4g of the lipase Rhizopus Niveus (Amano) was added and gently stirred. The adsorption of lipase was monitored by loss of activity from solution by assay. After 24 hours the polymer had adsorbed 92.7% of the lipase activity giving a theoretical loading of 9,270 LU/g polymer. The polymer was removed by filtration, washed twice with phosphate buffer (200 ml) and dried under vacuum at room temperature.

iii) Interesterification

The above catalyst was assessed for interesterification activity as follows: The sample (100 mg) was placed in a reaction vial and a solution containing 0.5g olive oil, 0.129g myristic acid and 10.0 ml petroleum ether (bp 100-120°C) added. The vial was sealed and placed in a waterbath (with shaker) at 40°C. Samples were taken after 1 hour and the amount of myristic acid incorporated into the triglyceride determined by FAME/GC analysis.

The results are shown in Table 4 together with results with Lipozyme catalyst (Mucor miehei lipase on an ion-exchange resin, from Novo). The Novo Lu Assay is a conventional standard assay to determine enzyme activity.

TABLE 4

| Catalyst | Interesterification (% myristic acid incorporated) | Novo Lu Assay ($\mu$moles/min/g) |
|---|---|---|
| Polystyrene | 7.9 | 2238 |
| Lipozyme | 10.6 | 557 |

EXAMPLE 5

i) Immobilization

To hydrophobic macroporous polystyrene particles 5693-93 (2.0g) was added 30 ml ethanol with stirring to ensure all the polymer particles were wetted. To this was added 200 ml 0.1M sodium phosphate buffer (pH7) with stirring to ensure complete mixing. Excess solution was decanted off and a further quantity of 200 ml buffer containing 47.5 ml Mucor miehei lipase solution (Lipozyme, Novo) was added. The solution was gently stirred. The adsorption of lipase by the porous polymer was monitored by loss of activity from the solution.

After 167 hours the support had adsorbed 68% of the lipase activity giving a theoretical loading of 173,100 LU/g. The catalyst was removed by filtration, washed twice with 0.1M phosphate buffer (200 ml), and dried

7

EP 0 322 213 B1

under vacuum at room temperature.

ii)Interesterification

A mixture of the catalyst (0.25g) and the same support (0.705g) without lipase was packed into a column 15 mm in diameter together with 4g wet ID silica gel (Crosfields) containing 3.2g water as a pre-column. Water-saturated feedstock comprising 1 part high oleate sunflower oil, 0.7 parts lauric acid and 4 parts petroleum ether (bp 100-120°C) by weight was pumped through the column which was maintained at 50°C using a water jacket The amount of lauric acid incorporated into the triglycerides was determined by FAME/GC analysis. The initial activity was calculated from flow rate and degree of conversion and was found to be 8.5g triglyceride processed/hour/g catalyst (this calculation is explained at the end of this specification).

EXAMPLE 6

i) Immobilization

To hydrophobic macroporous polystyrene particles 78-7300 (2.0g) was added 35 ml ethanol with stirring to ensure all the polymer particles were wetted. To this was added 200 ml 0.1M sodium phosphate buffer (pH7) with stirring to ensure complete mixing. Excess solution was decanted off and a further quantity of 200 ml buffer containing 20 ml Mucor miehei lipase solution (Lipozyme, Novo) added. The solution was gently stirred. The adsorption of lipase by the porous polymer was monitored by loss of activity from the solution. After 47.5 hours the support had adsorbed 85% of the lipase activity giving a theoretical loading of 92,600 LU/g. The catalyst was removed by filtration, washed twice with 0.1M phosphate buffer (200ml) and dried under vacuum at room temperature.

ii) Interesterification

A mixture of the catalyst (0.25g) and the same support (0.69g) without lipase was packed into a column 15 mm in diameter together with 4g wet ID silica gel (Crosfields) containing 3.2g water as a pre-column. Water-saturated feedstock comprising 1 part high oleate sunflower oil, 0.7 parts lauric acid and 4 parts petroleum ether (bp 100-120°C) by weight was pumped through the column which was maintained at 50°C using a water jacket. The amount of lauric acid incorporated into the triglycerides was determined by FAME/GC analysis. The initial activity was normalised for flow rate and degree of conversion and was found to be 7.5g triglyceride processed/hour/g catalyst.

EXAMPLE 7

i) Immobilization

To hydrophobic macroporous polypropylene particles EP-100 (2.0g) was added 20 ml ethanol with stirring to ensure all the polymer particles were wetted. To this was added 200 ml 0.1M sodium phosphate buffer (pH7) with stirring to ensure complete mixing. Excess solution was decanted off and a further quantity of 500 ml buffer containing Mucor miehei lipase solution (Lipozyme, Novo) added and the solution gently stirred. The adsorption of lipase by the porous polymer was monitored by loss of activity from the solution. This was done for three different amounts of lipase solution. The amount of enzyme added and the theoretical loadings achieved are given in Table 5 below. The catalyst was removed by filtration, washed twice with 0.1M phosphate buffer (200 ml) and dried under vacuum at room temperature.

ii) Interesterification

A mixture of the catalyst and the same support without lipase (in varying ratios, depending on the activity of the supported enzyme - see Table 5) was packed into a column 15 mm in diameter together with 4g wet ID silica gel (Crosfields) containing 3.2g water as a pre-column. Watersaturated feedstock comprising 1 part high oleate sunflower oil, 0.7 parts lauric acid and 4 parts petroleum ether (bp 100-120°C) by weight was pumped through the column which was maintained at 50°C using a water jacket. The amount of lauric acid incorporated into the triglycerides was determined by FAME/GC analysis. The initial activity was normalised for flow rate and degree of conversion.

8

The results are given in the Table 5 below (under A).

iii) Esterification

The catalyst (50 mg) was placed in a vial and a water-saturated mixture containing oleic acid (5.88g, 92%, from BDH, UK) and octan-1-ol (2.70g, GPR, from BDH) was added. The vial was sealed and placed in a water-bath at 50°C and left for 1 hour with continuous shaking (200 strokes/min). A sample (0.20 ml) was removed and immediately eluted down a small alumina column (basic, activity 2) with diethyl ether together with a solution (0.20 ml) of petroleum ether (bp 60-80°C) containing methyl stearate (5 mg) as an internal standard. The diethylether was then removed by evaporation and replaced by petroleum ether (bp 60-80°C, 6m). The ratio of octyl oleate to methyl stearate was then determined by GLC. From this ratio the rate of ester formation was calculated and the results given in the Table 5 (under B).

TABLE 5

| Lipase solution | | Theoretical loading (LU/g) | Activity | |
|---|---|---|---|---|
| (ml) | (% adsorbed) | | A | B |
| 6.6 | 98.5 | 35,700 | 2.3 | 66 |
| 48.0 | 92.2 | 244,800 | 34.0 | 330 |
| 308.0 | 80.0 | 1,188,000 | 63.6 | 546 |
| A = Interestification activity, g triglyceride/hour/g catalyst. | | | | |
| B = Esterification activity, micromoles ester/hour/mg catalyst. | | | | |

EXAMPLE 8

i) Immobilization

To the hydrophobic macroporous polymer particles of Amberlite XE-305 (2.0g) was added 20 ml ethanol with stirring to ensure all the polymer particles were wetted. To this was added 200 ml 0.1M sodium phosphate buffer (pH7) with stirring to ensure complete mixing. Excess solution was decanted off and a further aliquot of 100 ml buffer containing 5.5 ml Mucor miehei lipase solution (Lipozyme, Novo) added and the solution gently stirred, The adsorption of lipase by the porous polymer was monitored by loss of activity from the solution. After 2 hours the support had adsorbed 75.7% of the lipase activity giving a theoretical loading of 21,600 LU/g. The catalyst was removed by filtration, washed twice with 0.1M phosphate buffer (200ml) and dried under vacuum at room temperature.

ii) Interesterification

A mixture of the catalyst (1.0g) and the same support (1.0g) without lipase was packed into a column 15 mm in diameter together with 4g wet ID silica gel (Crosfields) containing 3.2g water as a pre-column. Water-saturated feedstock comprising 1 part high oleate sunflower oil, 0.7 parts lauric acid and 4 parts petroleum ether (bp 100-120°C) by weight was pumped through the column which was maintained at 50°C using a waterjacket. The amount of lauric acid incorporated into the triglycerides was determined by FAME/GC analysis. The initial activity was normalised for flow rate and degree of conversion and was found to be 2.0g triglyceride processed/hour/g catalyst.

EXAMPLE 9

i) Immobilization

To hydrophobic macroporous polymer particles EP-100 (2.0g) was added 20 ml ethanol with stirring to ensure all the polymer particles were wetted. To this was added 200 ml 0.1M sodium phosphate buffer (pH7) with stirring to ensure complete mixing. Excess solution was decanted off and a further quantity of 200 ml buffer containing 37.0g Rhizopus niveus lipase solution (Lipase N, Amano) added. The solution gently stirred. The adsorption of lipase by the porous polymer was monitored by loss of activity from the solution. After 22 hours the support had adsorbed 90% of the lipase activity giving a theoretical loading of 38,400 LU/g. The catalyst was removed by filtration, washed twice with 0.1M phosphate buffer (200 ml) and dried under vacuum at room temperature.

ii) Interesterification

A mixture of the catalyst (0.25g) and the same support (0.36g) without lipase was packed into a column 15 mm in diameter together with 4g wet ID silicagel (Crosfields) containing 3.2g water as a pre-column. Water-saturated feedstock comprising 1 part high oleate sunflower oil, 0.7 parts lauric acid and 4 parts petroleum ether (bp 100-120°C) by weight was pumped through the column which was maintained at 50°C using a water jacket. The amount of lauric acid incorporated into the triglycerides was determined by FAME/GC analysis. The initial activity was normalised for flow rate and degree of conversion and was found to be 35.0 g triglyceride processed/hour/g catalyst.

EXAMPLE 10

i) Immobilization

To hydrophobic macroporous polymer particles EP-100 (2.0g) was added 20 ml ethanol with stirring to ensure all the polymer particles were wetted. To this was added 200 ml 0.1M sodium phosphate buffer (pH7) with stirring to ensure complete mixing. Excess solution was decanted off and a further aliquot of 200 ml buffer containing 2 ml Humicola sp. lipase solution (SP398/PPW2542, Novo) added. The solution gently stirred. The adsorption of lipase by the porous polymer was monitored by loss of activity from the solution. After 2 hours the support had adsorbed 97% of the lipase activity giving a theoretical loading of 61,700 LU/g. The catalyst was removed by filtration, washed twice with 0.1M phosphate buffer (200 ml) and dried under vacuum at room temperature.

ii) Interesterification

A mixture of the catalyst (0.5g) and the same support (0.465g) without lipase was packed into a column 15 mm in diameter as a pre-column. Watersaturated feedstock comprising 1 part high oleate sunflower oil, 0.7 parts lauric acid and 4 parts petroleum ether (bp 100-120°C) by weight, was pumped through the column which was maintained at 50°C using a water jacket. The amount of lauric acid incorporated into the triglycerides was determined by FAME/GC analysis. The initial activity was normalised for flow rate and degree of conversion and was found to be 5.8g triglyceride processed/hour/g catalyst.

iii) Esterification

The same catalyst (50 mg) was placed in a vial and a water-saturated mixture containing oleic acid (5.88g, 92%, BDH) and octan-1-ol (2.70g, GPR, BDH) was added. The vial was sealed and placed in a water-bath at 50°C and left for 1 hour with continuous shaking (200 strokes/min). A sample (0.20 ml) was removed and immediately eluted down a small alumina column (basic, activity 2) with diethyl ether together with a solution (0.20 ml) of petroleum ether (bp 60-80°C) containing methyl stearate (5 mg) as an internal standard. The diethyl ether was then removed by evaporation and replaced by petroleum ether (bp 60-80°C, 6 ml). The ratio of octyl oleate to methyl stearate was then determined by GLC. From this ratio the rate of ester formation was calculated and was found to be 20 micromoles/hour/mg catalyst.

EXAMPLE 11

i) Silanisation

Controlled-pore glass beads PG-2000-120 (3.0g) were dried in an oven at 105°C for 15 minutes. When cool, a solution of dimethyldichlorosilane (6.7 ml) in 1,1,1-trichloroethane (27 ml) was added and the beads stirred. After 1.25 hours, the beads were filtered, washed with 1,1,1-trichloroethane and dried under vacuum at room temperature.

ii) Immobilisation

The silanised beads (3.0g) were allowed to stand in ethanol (100 ml) for 16 hours to ensure that the support was wetted. An aliquot (50 ml) of ethanol was removed and replaced with 0.1M sodium phosphate buffer (50 ml) at pH7. After stirring for a few minutes, an aliquot (50 ml) of the buffer/ethanol solution was withdrawn and an additional aliquot (50 ml) of phosphate buffer solution was added. Stirring was continued for a few more minutes before excess gas in the pores of the support was removed by application of a vacuum to the surface of the liquid. A final aliquot (50 ml) of the buffer/ethanol solution was then withdrawn, followed by the addition of a further aliquot (250 ml) of buffer solution containing Mucor miehei lipase solution (3.5 ml) (Lipozyme, Novo). The solution was gently stirred. The adsorption of lipase by the porous glass beads was monitored by the loss of activity from the solution. After 25.75 hours the beads had adsorbed 91.7% of the lipase activity to give a theoretical enzyme loading of 11,774 LU/g. The catalyst was isolated by filtration, washed with an aliquot (400 ml) of 0.1M sodium phosphate buffer solution, and then dried under vacuum at room temperature.

iii) Interesterification

The catalyst (3.0g) was packed into a column 15 mm in diameter together with 4g wet ID silica gel (Crosfields) containing 3.2g water as a pre-column. Water-saturated feedstock comprising 1 part high oleate sunflower oil, 0.7 parts lauric acid and 4 parts petroleum ether (bp 100-120°C) by weight, was pumped through the column which was maintained at 50°C using a water jacket. The amount of lauric acid incorporated into the triglycerides was determined by FAME/GC analysis. The initial activity was normalised for flow rate and degree of conversion and was found to be 1.3 g triglyceride processed/hour/g catalyst.

Activity calculation (used in Examples 5 to 11)

$$Activity = \frac{-\ln(1-DC) \times Flowrate}{wt.\ catalyst\ (g)}$$

Flowrate = g Triglyceride/hour through column
Degree of conversion (DC) =

$$\frac{\%\ incorporation\ lauric - \%\ initial\ content}{\%\ equilibrium\ content - \%\ initial\ content}$$

In this work, initial lauric content = 0
equilibrium content = 31.3%

**Claims**

1.  A process for the preparation of fatty acid esters, or for the re-arrangement of fats and vegetable oils, wherein reactants providing fatty acid and alcohol residues are reacted in a substantially non-aqueous, homogeneous liquid phase containing maximum 1 wt% of water in the presence of a supported lipase catalyst obtained by physically attaching the lipase by adsorption to the support material, characterized in that the support for the lipase catalyst is selected from the group consisting of 1) polyalkylenes, selected from the group consisting of homo- or copolymers of ethylene, styrene, butadiene or isoprene and homopolymers of propylene and 2) inorganic porous materials whose surfaces have been treated to render them hydrophobic, which support material has an average pore size from 0.05-5 μm.

2.  Process according to claim 1, wherein said reactants comprise glyceride esters.

3.  Process according to claim 2, wherein said glyceride esters comprise at least one of a fat and an oil.

**4.** Process according to claim 2 or claim 3, wherein the reactants include at least one free fatty acid or at least one alkyl ester of a free fatty acid.

**5.** Process according to claim 4, wherein the porous polymeric material has a total pore volume of at least 90%.

**6.** Process according to claim 1, wherein the support material comprises silica or glass with a surface of a hydrophobic organic silica polymer, obtained after a treatment of the support with a silane.

**Patentansprüche**

**1.** Verfahren zur Herstellung von Fettsäureestern oder zur Umlagerung von Fetten und pflanzlichen Ölen, wonach die Fettsäure- und Alkoholreste liefernden Reaktanten in einer im wesentlichen nichtwäßrigen homogenen flüssigen Phase, die maximal 1 Gew.-% Wasser enthält, in Gegenwart eines Lipaseträgerkatalysators umgesetzt werden, der durch physikalisches Binden der Lipase durch Adsorption an den Träger erhalten ist, dadurch gekennzeichnet, daß der Träger für den Lipasekatalysator aus der Gruppe ausgewählt ist, die aus 1) Polyalkylenen, ausgewählt aus der aus Homo- oder Copolymeren von Ethylen, Styrol, Butadien oder Isopren und Homopolymeren von Propylen bestehenden Gruppe, und 2) anorganischen porösen Materialien, deren Oberflächen zwecks Hydrophobierung behandelt worden sind, besteht, und das Trägermaterial eine durchschnittliche Porengröße von 0,05 bis 5 $\mu$m hat.

**2.** Verfahren nach Anspruch 1, wonach die Reaktanten Glyceridester umfassen.

**3.** Verfahren nach Anspruch 2, wonach die Glyceridester mindestens einen eines Fettes und eines Öles umfassen.

**4.** Verfahren nach Anspruch 2 oder 3, wonach die Reaktanten mindestens eine freie Fettsäure oder mindestens einen Alkylester einer freien Fettsäure umfassen.

**5.** Verfahren nach Anspruch 4, wonach das poröse polymere Material ein Porengesamtvolumen von mindestens 90 % aufweist.

**6.** Verfahren nach Anspruch 1, wonach des Trägermaterial Siliciumdioxid oder Glas mit einer Oberfläche aus einem hydrophoben organischen Siliciumdioxidpolymer umfaßt, erhalten nach Behandlung des Trägers mit eine Silan.

**Revendications**

**1.** Un procédé pour la préparation d'esters d'acides gras ou pour le réarrangement (interestérification) de matières grasses et huiles végétales, dans lequel on fait réagir des composants apportant des radicaux d'acides gras et d'alcools en phase liquide homogène pratiquement non aqueuse, contenant au maximum 1 % en poids d'eau, en présence d'un catalyseur consistant en une lipase sur support, obtenu par fixation physique de la lipase, par adsorption, sur la matière de support, caractérisé en ce que le support de la lipase servant de catalyseur est choisi dans le groupe consistant en 1) les polyalkylènes, eux-mêmes choisis parmi les homopolymères ou copolymères de l'éthylène, du styrène, du butadiène ou de l'isoprène et les homopolymères du propylène et 2) les matières poreuses minérales dont les surfaces ont été traitées en vue de les rendre hydrophobes, ladite matière de support ayant une dimension de pore moyenne de 0,05 à 5 $\mu$m.

**2.** Procédé selon la revendication 1, dans lequel lesdits composants comprennent des esters glycéridiques.

**3.** Procédé selon la revendication 2, dans lequel lesdits esters glycéridiques comprennent au moins un ester glycéridique choisi parmi les matières grasses et les huiles.

**4.** Procédé selon la revendication 2 ou 3, dans lequel les composants comprennent au moins un acide gras libre ou au moins un ester alkylique d'un acide gras libre.

**5.** Procédé selon la revendication 4, dans lequel la matière polymère poreuse a un volume de pores total d'au moins 90 %.

**6.** Procédé selon la revendication 1, dans lequel la matière de support consiste en silice ou en verre à surface consistant en un polymère organique silicié hydrophobe, obtenue par traitement du support à l'aide d'un silane.